# FASCICULE DE BREVET EUROPEEN

(11) **EP 2 836 180 B1**
(45) Date de publication et mention de la délivrance du brevet: **23.08.2017**
(21) Numéro de dépôt: 13722485.3
(22) Date de dépôt: 12.04.2013
(51) Int. Cl.: A61F 13/36

(54) **COMPRESSE LONGIFORME POUR USAGE MÉDICAL**
LÄNGLICHE KOMPRESSE ZUR MEDIZINISCHEN VERWENDUNG
ELONGATED PAD FOR MEDICAL USE

(30) Priorité: 13.04.2012 FR 1201116
(43) Date de publication de la demande: 18.02.2015
(73) Titulaire: Laboratoire Tetra Medical, 07100 Annonay (FR)
(72) Inventeur: MARCHAL, François, F-07370 Sarras (FR); ARTUS, Denis, F-40160 Parentis En Born (FR); BOUET, Thierry, F-07100 Annonay (FR)
(74) Mandataire: Jacobacci Coralis Harle
(86) Numéro de dépôt international: PCT/FR2013/050804
(87) Numéro de publication internationale: WO 2013/153343

(56) Documents cités:
- EP-A2- 1 256 330
- WO-A1-2004/110322

## Description

La présente invention concerne un dispositif longiforme absorbant destinés aux actes de chirurgie mini invasive, utilisant des trocarts.

Dans la plupart des opérations chirurgicales traditionnelles (ouvertes) ou par accès endoscopique, les opérateurs sont amenés à stopper des saignements.

Ils peuvent pour cela utiliser des clips, des instruments électriques de coagulation, des appareils d'irrigation /aspiration, ou enfin des compresses chirurgicales.

Les compresses sont le dispositif de choix car elles sont conformables, une capacité élevé d'absorption par rapport à leur poids et leur surface, et peuvent être laissées en place pendant un temps pour séparer des structures anatomiques.

Dans le cas précis des nombreuses interventions par trocarts, une compresse classique, pliée n'est pas adaptée car elle est très difficile à introduire dans le trocart. L'opérateur prend en outre le risque de déchirer le dispositif.

Des objectifs similaires ont été poursuivis par différentes inventions qui proposent des moyens de réalisations différents.

Le brevet de R. Seminara US 2007/0049860 décrit une éponge. Il se distingue de la présente invention en ce que le matériau retenu est uniquement mousse alvéolaire montée sur un manche. L'invention n'est pas uniquement destinée à l'endochirurgie. L'utilisation est différente d'une compresse. L'objet n'est pas introduit complètement dans l'intérieur du corps.

Le brevet de Microtech WO 2005/053589 décrit un matériau tubulaire hémostatique, type mousse de PVA. Celui-ci est de nature tampon absorbant d'une seule forme. La présente invention se distingue par le fait qu'une partie de matériau absorbant peut se déplier comme une compresse absorbante. Le dispositif décrit est associé à un instrument spécifique d'introduction. De plus le procédé de récupération nécessite une extrémité d'instrument adaptée, mais aussi un certain entrainement au geste.

Le brevet de I. YOON WO 92/01433 décrit un dispositif conçu pour la chirurgie endoscopique, basé sur un instrument. Mais le consommable est nécessairement adapté à un seul instrument. Il permet l'absorption, l'irrigation et l'aspiration, mais comme d'autres inventions n'est pas prévu pour être introduit complètement dans la cavité intérieure. Le matériau absorbant reste toujours lié au trocart.

Le brevet USSC n°5522795 décrit lui aussi un dispostif absorbant pour la chirurgie endoscopique. Cette invention, un peu comme celle d'YOON précédemment citée, est composé « d'un matériau flexible » une compresse de petite dimension d'où faible capacité d'absorption, intimement lié au dispositif introducteur. A aucun moment, le petit tampon absorbant fixé à sa partie tubulaire n'est prévu pour être détaché et récupéré contrairement à la présente invention.

Enfin un autre brevet proche de la présente application, Shlain LEONARD, daté de 1992, soit un an avant le brevet USSC, décrit aussi un ruban souple absorbant sortant d'un guide introducteur. Ce matériau reste attaché à l'instrument et est tributaire d'un dispositif spécifique d'introduction.

On connait également par le document WO2004/110322 une compresse repliée sur elle-même et cousue le long de ses quatre cotés et qui est de ce fait de forme rectangulaire comme représenté sur sa Figure 7 de test de passage dans un trocart. Un fil radio opaque est également fixé sur la compresse.

L'objet du brevet est donc un dispositif réunissant les caractéristiques d'une compresse et une forme adaptée à l'introduction et au retrait aisé par la lumière des trocarts de chirurgie. Le dispositif est constitué d'un matériau fibreux ou spongieux souple enroulé ou replié, ces termes étant ici considérés équivalents dans le contexte de l'invention, dans une dimension (une extrémité de largeur en l'espèce) pour former un cylindre de diamètre inférieur au diamètre intérieur des trocarts usuels et qui peut se dérouler ou se déplier en partie une fois sorti du trocart pour avoir une surface de contact augmentée. Plus particulièrement la compresse qui est de forme rectangulaire avec deux extrémités de largeur et deux côtés de longueur est enroulée ou repliée sur une (ou les deux) de ses deux extrémités de largeur et cet enroulement ou repliement est fixé par un moyen de fixation qui ne concerne donc que ladite extrémité de largeur. De ce fait, le reste de la compresse reste libre de se déployer.
Plus précisément, l'invention concerne une compresse longiforme absorbante comportant deux extrémités de largeur opposées, ladite compresse étant constituée d'un matériau hydrophile, souple, et possédant une partie détectable de l'extérieur du corps.

Selon l'invention, la compresse comporte au moins une de ses deux extrémités de largeur repliée sur elle-même, ledit repliement de ladite extrémité de largeur étant maintenu par un moyen de fixation selon un repliement réalisant une forme cylindroïde de la compresse et permettant l'introduction et le retrait dans un trocart.

Dans divers modes de mise en oeuvre de l'invention, les moyens suivants pouvant être utilisés seuls ou selon toutes les combinaisons techniquement possibles, sont employés :
- la compresse utilise un textile non déformable dans la plus grande longueur de l'objet, et ayant une vitesse d'absorption équivalente aux compresses chirurgicales,
- la compresse est fabriquée dans un matériau très absorbant et rétenteur de liquide, biocompatible et stérilisable,
- le moyen de fixation pour maintenir le repliement de l'extrémité de largeur sur elle-même est choisi parmi : une couture, un manchon plastique serré, une soudure Haute Fréquence ou Ultra-son ou un collage ou un adhésif enroulé serré,
- la compresse est munie d'une attache souple,
- l'attache souple est de longueur variable,
- l'attache souple est élastique,
- l'attache souple est fixée par le moyen de fixation à l'extrémité de largeur repliée sur elle-même,
- la surface dépliée, c'est-à-dire celle qui n'est pas fixée par le moyen de fixation, de matériau absorbant soit définie pour que le repliement constitue une forme cylindrique de dimension compatible avec les trocarts habituellement utilisés en chirurgie,
- le matériau absorbant et souple possède des bords soudés ou ourlés renforcés non effritables ou effilochables empêchant le relargage de particules,
- le matériau plié présente une surface réduite, et le déploiement de la partie du matériau en dehors de l'extrémité de largeur repliée sur elle-même et maintenue par le moyen de fixation présente une surface importante permettant de former une barrière sur le site opératoire.

La présente invention, sans qu'elle en soit pour autant limitée, va maintenant être exemplifiée avec la description qui suit de modes de réalisation et de mise en oeuvre en relation avec :
la Figure 1 qui représente la compresse dont une extrémité de largeur repliée et fixée par un moyen de fixation a été cousue lui donnant une forme cylindroïde ou, plus précisément, conique / en éventail comme représenté, sa partie non cousue étant libre de se déployer,
la Figure 2 qui représente la compresse prise à son extrémité de largeur cousue pour introduction dans un trocart ;
la Figure 3 qui représente la compresse après son utilisation pour retrait à travers le trocart, et
la Figure 4 qui représente une variante de réalisation de la compresse comportant une attache.

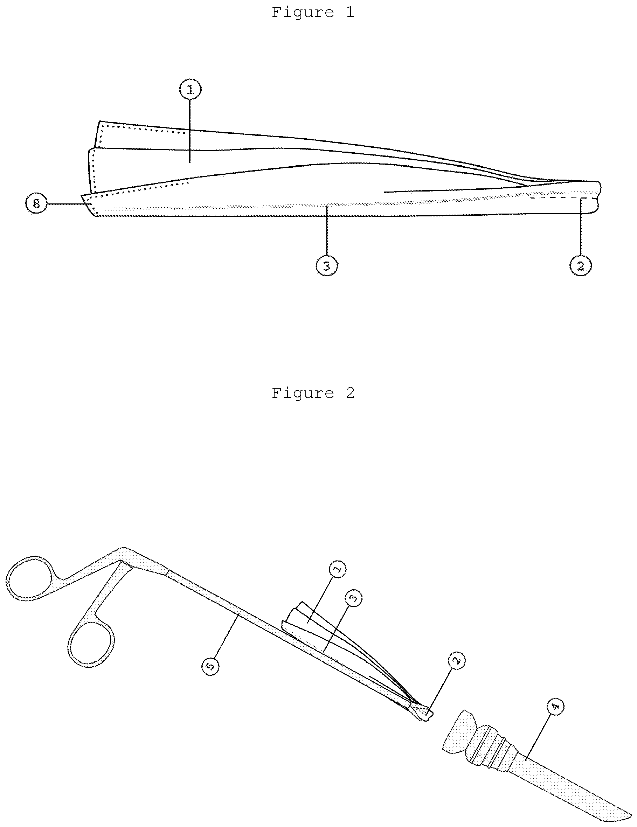

Un mode de réalisation préféré est l'obtention d'un corps longiforme souple, et déformable comme représenté figure 1. La caractéristique déformable permet de saisir le dispositif par une pince d'endochirurgie 5 pour l'introduction et la traction dans le trocart comme représenté figure 2, de telle sorte que le corps absorbant et les mors de la pince ensembles aient un encombrement inférieur à la lumière du trocart.

Pour absorber la plupart des matériaux hydrophiles et souples peuvent convenir. Ils doivent présenter une grande capacité d'absorption en masse d'eau absorbée par rapport à la masse de la compresse sèche, à titre d'exemple au moins 8 grammes par gramme. Le matériau peut être un polymère alvéolaire (type mousse de polyuréthanne), un non-tissé épais (type feutre de polyester, viscose ou autre polymère hydrophile) ou un tissu chaine/trame ou encore un tricot.

Un mode préférentiel de réalisation utilise un non tissé souple et résistant, présentant aussi une surface dépliée importante. Le matériau est rétenteur de liquide et stérilisable.

La résistance et la déformation du matériau choisi peuvent être différentes selon le sens de traction. Même si l'extraction par le trocart est rendue aisée par la forme adaptée de la compresse, celle-ci doit présenter une résistance minimum de 30 Newtons par cm et une faible déformation (< 15% d'allongement à la rupture). La compresse doit être non déformable dans la plus grande longueur de l'objet.

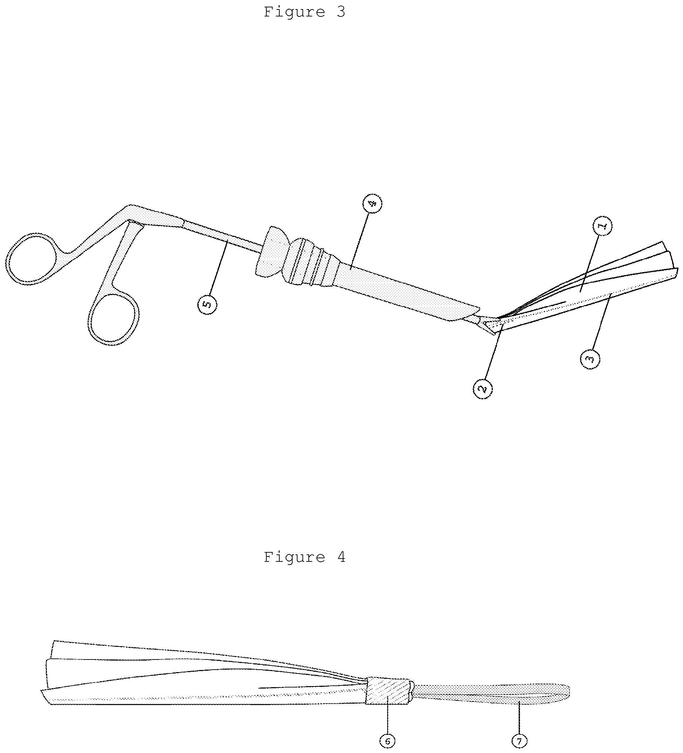

Le mode de liaison/fixation du matériau replié à au moins une de ses deux extrémités de largeur peut être une couture, une soudure haute fréquence ou ultra-son, un collage ou l'application d'un manchon plastique serré ou enfin l'application d'un adhésif enroulé serré comme représenté figure 4. Tout autre moyen de fixation du matériau absorbant sur lui-même, par exemple agrafe, est aussi possible. Le matériau plié présente une surface réduite, et le déploiement de la partie non cousue, dépliée présente une surface importante permettant de former une barrière sur le site opératoire. Le fait qu'une partie de matériau absorbant peut se déplier comme représenté figure 1 est important pour pouvoir, une fois déployé constituer une barrière temporaire. Autrement dit la fixation par le moyen de fixation, couture ou autre, ne porte pas sur toute la longueur de la compresse ni sur tout le pourtour de la compresse mais sur seulement un bord replié de la largeur de la compresse comme représenté. De ce fait, la compresse avec son extrémité de largeur, replié et fixé présente une forme cylindroïde qui s'apparente à un cône ou éventail. Dans une variante non représentée, chacune des deux extrémités de largeur sont repliées et fixées. La longueur de la fixation en mode replié est déterminée par la souplesse que l'on veut obtenir pour l'ensemble. Le matériau choisi doit aussi être biocompatible et compatible avec un mode de stérilisation pour être utilisé au bloc opératoire.

La compresse possède en outre une partie détectable de l'extérieur du corps ce qui, dans le contexte de l'invention, signifie que la compresse peut être détectée par un équipement de radiologie ou d'ultrasons voire un détecteur de métaux à partir de l'extérieur du corps.

Le dispositif peut être prolongé par une attache textile ou plastique saisissable par un instrument type pince comme représenté figure 4. Ainsi, la compresse peut être munie d'une attache souple 7 de longueur variable.

Le matériau retenu doit présenter aussi une vitesse d'absorption en rapport avec le besoin du geste chirurgical (par exemple temps d'immersion < 10 secondes).

La surface de matière absorbante est définie pour que le repliement constitue une forme cylindrique de dimension compatible avec les trocarts habituellement utilisé en chirurgie, à titre d'exemple non limitatif, de l'ordre de 15mm de diamètre pour les plus larges jusqu'à 5 mm de diamètre pour les trocarts fins.

Le matériau absorbant ne doit pas présenter de risque d'effilochage ou d'effritement de particules ou fibres sur les bords lorsque imprégné de liquide ou s'il est saisi par les mors d'un instrument. Dans le cas d'un matériau fibreux, les bords de chaque cotés pourront être traités thermiquement (thermo fusion ou fixation). Dans le cas d'un matériau tissé ou tricotée, une piqure couture type surjet ou ourlé pourra renforcer l'objet. Un mode de réalisation pourra être des bords ourlés renforcés, soudés empêchant le relargage de particules comme schématisé en 8 sur la figure 1.

Finalement, l'invention du présent document, présente l'avantage d'utiliser des instruments de chirurgie endoscopique existants, et ne nécessite pas d'apprentissage spécifique dans son utilisation. Lors de l'extraction de la compresse, figure 3, de la cavité endochirurgicale, si la préhension de la compresse 1 par la pince 5 n'est pas dans l'axe du trocart 4, la souplesse du matériau permet néanmoins à l'extrémité proximale de la compresse de s'adapter à la lumière du trocart. La compresse est introduite le plus souvent sèche et retirée humide, donc une taille plus petite que le diamètre intérieur de trocart favorisera la rétention de liquide absorbé dans le site opératoire.

## Revendications

1. Compresse (1) longiforme absorbante comportant deux extrémités de largeur opposées, ladite compresse étant constituée d'un matériau hydrophile, souple, et possédant une partie détectable (3) de l'extérieur du corps, **caractérisée en ce qu'**elle comporte au moins une de ses deux extrémités de largeur repliée sur elle-même, ledit repliement de ladite extrémité de largeur étant maintenu par un moyen de fixation (2) selon un repliement réalisant une forme cylindroïde de la compresse et permettant l'introduction et le retrait dans un trocart, et **en ce que** le matériau plié présente une surface réduite, et le déploiement de la partie du matériau en dehors de l'extrémité de largeur repliée sur elle-même et maintenue par le moyen de fixation (2) présente une surface importante permettant de former une barrière sur le site opératoire.

2. Compresse selon la revendication 1, **caractérisée en ce qu'**elle utilise un textile non déformable dans la plus grande longueur de l'objet, et ayant une vitesse d'absorption équivalente aux compresses chirurgicales.

3. Compresse selon l'une des revendications 1 ou 2, **caractérisée en ce qu'**elle est fabriquée dans un matériau très absorbant et rétenteur de liquide, biocompatible et stérilisable.

4. Compresse selon l'une des revendications précédentes, **caractérisée en ce que** le moyen de fixation (2) pour maintenir le repliement de l'extrémité de largeur sur elle-même est choisi parmi: une couture, un manchon plastique serré, une soudure Haute Fréquence ou Ultra-son ou un collage ou un adhésif enroulé serré (6).

5. Compresse selon l'une des revendications ci-dessus, **caractérisée en ce qu'**elle est munie d'une attache souple.

6. Compresse selon la revendication 5, **caractérisée en ce que** l'attache souple est de longueur variable (7).

7. Compresse selon la revendication 5 ou 6, **caractérisée en ce que** l'attache souple est fixée par le moyen de fixation (2) à l'extrémité de largeur repliée sur elle-même.

8. Compresse selon l'une des revendications précédentes, **caractérisée en ce que** la surface dépliée de matériau absorbant soit définie pour que le repliement constitue une forme cylindrique de dimension compatible avec les trocarts habituellement utilisés en chirurgie.

9. Compresse selon l'une des revendications précédentes, **caractérisée en ce que** le matériau absorbant et souple possède des bords soudés ou ourlés renforcés non effritables ou effilochables empêchant le relargage de particules (8).

## Patentansprüche

1. Längliche absorbierende Kompresse (1) mit zwei voneinander abgewandten breitseitigen Enden, wobei die Kompresse aus einem hydrophilen weichen Material besteht und einen von außerhalb des Körpers erkennbaren Teil (3) aufweist, **dadurch gekennzeichnet, daß** wenigstens eins ihrer beiden breitseitigen Enden auf sich selbst zurückgefaltet ist, wobei die Zurückfaltung des breitseitigen Endes durch ein Mittel (2) zur Befestigung durch eine Zurückfaltung aufrechterhalten wird, die eine zylindroide Form der Kompresse erzeugt und das Einführen in und das Herausnehmen aus einem Trokar ermöglicht, und dadurch, daß das gefaltete Material eine reduzierte Oberfläche aufweist und daß die Ausbreitung des Teils des Materials außerhalb des auf sich selbst zurückgefalteten und durch das Befestigungsmittel (2) festgehaltenen breitseitigen Endes eine große Oberfläche aufweist, die ermöglicht, eine Sperre an einer Operationsstelle zu bilden.

2. Kompresse gemäß Anspruch 1, **dadurch gekennzeichnet, daß** sie einen in der größten Länge des Gegenstands nicht verformbaren und eine chirurgischen Kompressen gleichwertige Absorptionsgeschwindigkeit aufweisenden Stoff verwendet.

3. Kompresse gemäß einem der Ansprüche 1 oder 2, **dadurch gekennzeichnet, daß** sie aus einem stark absorbierenden, Flüssigkeit zurückhaltenden, biokompatiblen und sterilisierbaren Material gefertigt ist.

4. Kompresse gemäß einem der vorangehenden Ansprüche, **dadurch gekennzeichnet, daß** das Befestigungsmittel (2) zum Aufrechterhalten der Zurückfaltung des breitseitigen Endes auf sich selbst aus einer Naht, einer engen Plastikhülse, einer Hochfrequenz- oder Ultraschallschweißung oder einer Klebung oder einem eng gewickelten Klebeband ausgewählt ist.

5. Kompresse gemäß einem der vorangehenden Ansprüche, **dadurch gekennzeichnet, daß** sie eine nachgiebige Befestigung aufweist.

6. Kompresse gemäß Anspruch 5, **dadurch gekennzeichnet, daß** die nachgiebige Befestigung eine veränderbare Länge (7) aufweist.

7. Kompresse gemäß Anspruch 5 oder 6, **dadurch gekennzeichnet, daß** die nachgiebige Befestigung durch das Befestigungsmittel (2) an dem auf sich selbst zurückgefalteten breitseitigen Ende befestigt ist.

8. Kompresse gemäß einem der vorangehenden Ansprüche, **dadurch gekennzeichnet, daß** die ausgebreitete Oberfläche absorbierenden Materials so definiert ist, daß die Zurückfaltung eine zylindrische Form mit mit den in der Chirurgie üblicherweise verwendeten Trokars kompatiblen Abmessungen bildet.

9. Kompresse gemäß einem der vorangehenden Ansprüche, **dadurch gekennzeichnet, daß** das absorbierende und weiche Material verschweißte oder umgeschlagene verstärkte, nicht zerkrümelnde oder fusselnde Ränder aufweist, die ein Freisetzen von Teilchen (8) verhindern.

## Claims

1. An absorbent elongated pledget (1) having two opposite width ends, said pledget being made from a flexible, hydrophilic material, and having a portion that can be detected from outside the body (3), **characterized in that** it has at least one of its two width ends that is folded over itself, said fold of said width end being held by a fastening means (2) in a fold forming a cylindroid shape of the pledget and allowing the insertion into and removal from a trocar, and **in that** the folded-over material has a reduced surface, and the opening out of the portion of material outside the width end folded over itself and held by the fastening means (2) has a significant surface allowing to form a barrier on the operation site.

2. The pledget according to claim 1, **characterized in that** it uses a non-deformable fabric in the greatest length of the object, and having a speed of absorption equivalent to that of the surgical pledgets.

3. The pledget according to one of claims 1 or 2, **characterized in that** it is manufactured from a very absorbent and liquid retaining, biocompatible and sterilisable material.

4. The pledget according to one of the preceding claims, **characterized in that** the fastening means (2) for holding the fold of the width end over itself is chosen among: a seam, a press-fit plastic sleeve, a High-Frequency or ultrasonic weld, or a bond or a hard wound adhesive (6).

5. The pledget according to one of the above claims, **characterized in that** it is provided with a flexible fastener.

6. The pledget according to claim 5, **characterized in that** the flexible fastener is of variable length (7).

7. The pledget according to claim 5 or 6, **characterized in that** the flexible fastener is fastened by the fastening means (2) at the width end folded over itself.

8. The pledget according to one of the preceding claims, **characterized in that** the unfolded surface of absorbent material is defined so that the fold forms a cylindrical shape of dimension compatible with the trocars usually used in surgery.

9. The pledget according to one of the preceding claims, **characterized in that** the absorbent and flexible material has uncrumbable and unfrayable, reinforced, welded or hemmed edges preventing the salting out of particles (8).
